# FASCICULE DE BREVET EUROPEEN

(11) **EP 1 148 791 B1**
(45) Date de publication et mention de la délivrance du brevet: **11.12.2002**
(21) Numéro de dépôt: 99952602.3
(22) Date de dépôt: 14.10.1999
(51) Int. Cl.: A23J 3/34, A23L 1/305, A23J 3/08, A23J 3/10, A23K 1/18

(54) **MATIERE PROTEIQUE A DIGESTION RALENTIE ET SON UTILISATION**
PROTEINMATERIAL MIT VERLANGSAMTEM VERDAUNGSABLAUF UND DEREN VERWENDUNG
PROTEIN MATERIAL FOR SLOW DIGESTION AND ITS USE

(30) Priorité: 16.10.1998 EP 98203452
(43) Date de publication de la demande: 31.10.2001
(73) Titulaire: SOCIETE DES PRODUITS NESTLE S.A., 1800 Vevey (CH)
(72) Inventeur: BALLEVRE, Olivier, CH-1000 Lausanne 25 (CH); GARCIA-RODENAS, Clara, L., CH-1606 Forel (CH); REIFFERS-MAGNANI, Kristel, CH-1814 La Tour-de-Peilz (CH); BEAUFRERE, Bernard, F-63400 Chamalières (FR); DANGIN, Martial, F-63000 Clermont-Ferrand (FR); COUZY, François, CH-1073 Savigny (CH)
(86) Numéro de dépôt international: EP9907909
(87) Numéro de publication internationale: WO00022937

(56) Documents cités:
- EP-A- 0 799 577
- WO-A-97/05785
- BOIRIE YVES; ET AL: "SLOW AND FAST DIETARY PROTEINS DIFFERENTLY MODULATE POSTPRANDIAL PROTEIN ACCRETION" PROC. NATIONAL ACADEMY OF SCIENCE, PHYSIOLOGY, vol. 94, décembre 1997 (1997-12), pages 14930-14935, XP002099786 USA cité dans la demande

## Description

L'invention a pour objet l'utilisation d'une matière protéique dont la vitesse de digestion a été diminuée pour la préparation d'une composition permettant de moduler le taux plasmatique postprandial en acides aminés. L'invention a également pour objet une composition destinée à être administrée par voie entérale à un mammifère contenant une matière protéique dont la vitesse de digestion à été ralentie.

### État de la technique

Du fait d'un besoin permanent en nutriments et de la nature périodique de l'alimentation chez l'Homme, l'organisme a du développer des processus de stockage des nutriments absorbés en excès pendant le repas et des mécanismes de mobilisation de ces réserves pendant la période de jeûne physiologique. L'alternance des périodes d'alimentation et de jeûne sont à l'origine de profondes modifications des différentes voies du métabolisme des nutriments.

Ces variations nycthémérales affectent la synthèse et la dégradation des protéines et par conséquent le bilan protéique. Ainsi, le bilan protéique négatif pendant la période de jeûne physiologique devient positif pendant la période postprandiale, phase d'assimilation des nutriments à partir du tube digestif. L'importance relative de chaque phase détermine alors l'évolution de la masse protéique corporelle. Il est donc essentiel de pouvoir améliorer le gain protéique postprandial afin d'optimiser l'évolution de la masse protéique.

L'ingestion de repas constitués de protéines provoque une augmentation du taux plasmatique en acides aminés. Cette élévation de la disponibilité en acides aminés est associée à un remaniement des différentes composantes du métabolisme protéique (dégradation protéique, synthèse protéique, oxydation des acides aminés). Récemment, Boirie et al (*Proc. Natl. Acad. Sci. USA,* **94,** 14930-14935, 1997) ont montré chez de jeunes volontaires sains, que le gain protéique postprandial dépendait de la vitesse de digestion des protéines ingérées (délai entre l'ingestion et l'absorption des nutriments par l'organisme).

Certaines protéines à vitesse de digestion rapide comme les protéines du lactosérum peuvent présenter une valeur nutritive élevée, c'est à dire un apport adéquat et équilibré en acides aminés essentiels pour l'organisme humain, comme la valine, leucine, isoleucine, phénylalanine, lysine, méthionine, tryptophane et thréonine. Toutefois, malgré ce bon équilibre en acides aminés, l'utilisation corporelle des acides aminés issus de ces protéines n'est pas optimum, puisqu'elles sont digérées trop rapidement. Aussi, le document WO 9705785, décrit une composition utilisée dans les aliments pour nouveaux-nés qui contient des protéines à digestion lente, lesdites protéines ayant été préalablement modifiées afin de ralentir la vitesse de digestion.

On peut alors utiliser d'autres sources contenant des protéines ayant une vitesse de digestion naturellement plus lente, comme les caséines, par exemple, mais dont l'apport et l'équilibre en acides aminés ne sont pas optimaux.

La présente invention vise à assurer les besoins nutritifs de certaines catégories de personnes, au moyen de protéines, dont la vitesse de digestion est diminuée.

### Résumé de l'invention

L'invention concerne ainsi l'utilisation d'une matière protéique à digestion lente pour la préparation d'une composition destinée à être administrée par voie entérale à un mammifère afin de moduler le taux plasmatique post-prandial en acides aminés, ladite matière protéique ayant été préalablement traitée de sorte à transformer les protéines à digestion rapide qu'elle contenait en protéines à digestion lente caractérisée en ce que la matière protéique à digestion lente est une matière contenant des protéines gélifiées microparticulées associées à des polysaccharides dans des conditions d'incompatibilité thermodynamique.

A ce jour, il n'a jamais été proposé de réduire la vitesse de digestion d'une protéine dans le but de moduler le taux plasmatique post-prandial en acides aminés afin :
a) d'accroître le gain protéique post-prandial, et/ou
b) d'éviter une surcharge métabolique de certains organes et/ou de certaines enzymes et/ou
c) de limiter la prise alimentaire quotidienne grâce à un effet satiétogène de ces protéines et/ou
d) de pallier à certains dysfonctionnements du métabolisme des acides aminés et plus spécifiquement à des déficits enzymatiques.
e) d'améliorer la régénération des tissus, notamment les processus de cicatrisation.

Ce traitement est particulièrement avantageux pour les protéines à haute valeur nutritionnelle et qui sont digérées trop rapidement, et ce de manière à optimiser le gain protéique.

L'invention a également pour objet une composition destinée à être administrée par voie entérale à un mammifère, contenant une matière protéique à digestion lente ayant été préalablement traitée de sorte à transformer les protéines à digestion rapide qu'elle contenait en protéines à digestion lente, caractérisée en ce que la matière protéique à digestion lente est une matière contenant des protéines gélifiées microparticulées associées à des polysaccharides dans des conditions d'incompatibilité thermodynamique.

Les compositions ainsi obtenues peuvent être particulièrement indiquées pour :
- minimiser les pertes de protéines corporelles chez les personnes âgées, les patients gravement malades ou les personnes suivant un régime basse calorie
- les patients atteints de troubles rénaux ou hépatiques
- les patients atteints de dysfonctionnements du métabolisme des acides aminés comme par exemple l'hyperphenylalaninemia ou autres aminoacidopathies
- les patients traités à la L-DOPA
- les prématurés.

Elles peuvent également être destinées à la nutrition des animaux familiers, en particulier celle des sujets âgés, des jeunes en période de croissance et pour contrôler le poids corporel de certains sujets.

### Description détaillée de l'invention

Dans le cadre de la présente invention, une matière protéique à digestion lente est une matière qui, fournie sous forme de solution et digérée par des rats de 140-200g, conduit à une disparition de la moitié de l'azote ingéré présent dans le tube digestif en plus de 80 min.

On appelle protéine rapide des protéines qui, lorsqu'elles sont ingérées sous forme de solution par des rats de 140-200g, conduisent à une disparition de la moitié de l'azote ingéré présent dans le tube digestif en moins de 70 min.

Pour mettre en oeuvre la présente invention, on utilise une matière protéique, c'est à dire toute matière comprenant des protéines, qu'elles soient d'origine animale, végétale ou microbienne, notamment des protéines de lait, d'oléagineux, de légumineuses, d'oeuf ou de levures de bière, par exemple.

Les matières contenant des protéines ayant une valeur nutritive élevée, selon les apports recommandés, sont particulièrement indiquées dans le cadre de la présente invention. Ces protéines peuvent contenir une teneur équilibrée et élevée en chacun des acides aminés essentiels pour le corps, comme la lysine, le tryptophane, la leucine, l'isoleucine, la valine, la phénylalanine, la méthionine et la thréonine, par exemple.

De préférence, la matière contenant des protéines (non traitée) comprend des protéines à digestion rapide, comme par exemple les protéines de lactosérum.

La matière contenant des protéines est traitée de façon à ce que la vitesse de digestion desdites protéines soit ralentie. A cet effet, la matière contenant des protéines est mélangée à des polysaccharides et, dans des conditions d'incompatibilité thermodynamique, forme des microparticules gélifiées par traitement thermique.

En effet, des biopolymères tels que les protéines et les polysaccharides, peuvent présenter une incompatibilité thermodynamique ; c'est à dire qu'en dessus d'une concentration seuil ils ne forment pas un mélange homogène et se séparent spontanément en deux phases. L'une est enrichie en protéines, l'autre est enrichie en polysaccharides. Au stade initial, la séparation des deux phases se fait par formation de gouttelettes microscopiques, qui peuvent être gélifiées ; dans le cas de gouttelettes protéiques, un traitement thermique permet souvent de former un gel. Ainsi la microparticulation protéique résulte d'une séparation de phase et d'une gélification simultanée d'un mélange aqueux de protéines et de polysaccharides (Syrbe, Ph.D Thesis, Techn. Univ. Munich, 1997).

Les polysaccharides selon la présente invention peuvent être notamment choisis parmi les alginates, la gomme xanthane, la gomme arabique, le guar, l'amidon, les maltodextrines et dextrines, les pectines, les kappa-carraghénanes, les iota-carraghénanes, les lambda-carraghénanes, la méthyl-cellulose et la carboxy-méthyl-cellulose, les dextranes sulphatés et/ou la gomme gellane.

Les concentrations en protéines et en polysaccharides dans le mélange peuvent être respectivement comprises entre 3 et 12% et entre 0,2 et 1%. Le rapport protéine/ polysaccharides pouvant varier ainsi de 3:1 à 24:1.

Les microparticules peuvent par exemple être préparées à partir d'un mélange d'une solution d'alginate et d'une solution de protéines sériques. La solution d'alginate est de préférence à 3% et pH 7 et la solution de protéines sériques à 15%, pH 6,6. Le mélange peut être alors chauffé à une température comprise entre 70 et 130°C pendant une durée de 1-60 minutes.

Les microparticules obtenues ont un diamètre de préférence compris entre 200 nm et 100 microns.

Les conditions de traitement de la matière contenant les protéines doivent être de préférence choisies de sorte à atteindre un niveau de ralentissement de la vitesse de digestion desdites protéines tel que lorsque la matière protéique traitée est administrée oralement sous forme de solution à des rats de 140-200g, elle conduit à une disparition de la moitié de l'azote ingéré présent dans le tube digestif en plus de 80 min, par exemple.

La matière protéique ainsi traitée peut être avantageusement utilisée pour la préparation d'une composition alimentaire ou pharmaceutique destinée à être administrée oralement à un mammifère afin
1. d'accroître le gain protéique post-prandial, et/ou
2. d'éviter une surcharge métabolique de certains organes et/ou de certaines enzymes et/ou
3. de limiter la prise alimentaire quotidienne grâce à un effet satiétogène de ces protéines et/ou
4. de pallier à certains dysfonctionnements du métabolisme des acides aminés et plus spécifiquement à des déficits enzymatiques et/ou
5. d'améliorer l'efficacité des traitements parla L-DOPA
6. d'améliorer la régénération des tissus, notamment les processus de cicatrisation.

Ainsi, certaines modifications technologiques, comme la gélification thermique, le mélange de ces protéines avec des polysaccharides pouvant gélifier dans l'estomac, la formation de microparticules gélifiées, ainsi que l'apport préalable de casomorphines sous forme d'un hydrolysat de caséine peuvent rendre plus lente la vitesse de digestion de protéines.
On peut par exemple utiliser une des matières contenant des protéines précitée, combinée avec des polysaccharides anioniques.

La matière protéique à vitesse de digestion lente est susceptible d'améliorer ou de prévenir des problèmes liés à des états physiologiques ou physiopathologiques variés. En fait, les matières protéiques à vitesse de digestion lente peuvent agir selon 4 modalités principales, en optimisant le gain protéique postprandial, en évitant une surcharge de fonctionnement pour des organes clés ou pour certaines enzymes, en optimisant les traitements avec de la L-DOPA et en augmentant la sensation de satiété. Les conditions régissant l'utilisation de ces protéines dépendront notamment des catégories de personnes concernées.

Dans le cadre de l'optimisation du gain protéique post-prandial, les cas de dénutrition peuvent être traités. La dénutrition se présente fréquemment chez les sujets âgés ou lors de maladies qui comportent une déperdition importante des protéines corporelles - insuffisance rénale, brûlure sévère, traumatisme, stress chirurgical ou infectieux, inflammation, cancers ou SIDA. Cet état métabolique se traduit par un bilan azoté négatif qui est la conséquence d'une fonte des protéines corporelles et plus particulièrement musculaires. En effet, les protéines musculaires sont dégradées afin de fournir de l'énergie à l'organisme et de permettre la redistribution des acides aminés vers la synthèse de protéines spécifiques.

Dans les cas de dénutrition, l'ingestion de matière protéique à vitesse de digestion lente est susceptible de limiter cette perte protéique, en optimisant le gain protéique postprandial. Cette matière protéique devrait augmenter la rapidité de la récupération physiologique, la résistance aux agressions, la qualité de vie et donc le pronostic vital.

L'anomalie rénale, au sens large du terme, est un exemple d'utilisation de la matière protéique à vitesse de digestion lente qui ne repose pas uniquement sur l'optimisation du gain protéique postprandial, bien qu'elle en soit une composante essentielle. En effet, lors d'anomalies rénales, les patients sont soumis à un régime hypoprotéique strict afin de diminuer la production de déchets azotés. Il est couramment admis qu'un tel régime a un effet favorable sur l'état général, la qualité de vie et même sur la fonction rénale. Cependant, ce régime alimentaire est très mal toléré par les malades. L'ingestion de matière protéique à vitesse de digestion lente contribue à :
1) diminuer la production d'azote qui doit être ultérieurement éliminé par les reins;
2) répartir cette production sur une durée beaucoup plus longue; et
3) augmenter l'action satiétogène de ce type de protéines pour assurer une meilleure tolérance du régime.
Les protéines à vitesse de digestion lente sont par conséquent particulièrement indiquées pour la nutrition des patients qui présentent des troubles rénaux.

De même, la matière protéique à vitesse de digestion lente peut être prescrites aux patients qui présentent des pathologies hépatiques. Après un repas composé de différents composés azotés (protéines, peptides, acides aminés), le foie va tenter de maintenir la concentration en acides aminés dans des limites physiologiques en dégradant une partie des acides aminés issus de l'alimentation. Une arrivée modérée des acides aminés alimentaires est susceptible de réduire la surcharge d'activité d'un organe qui présente des pathologies et par conséquent permettra d'éviter une surcharge de travail. En outre, la matière protéique à vitesse de digestion lente induit un meilleur gain protéique postprandial.

Lors de déficit en enzymes pancréatiques protéolytiques, l'ingestion de matière protéique à vitesse de digestion lente peut contribuer à l'amélioration du processus de digestion. Ce bénéfice est assuré par la diminution de la quantité de substrat à hydrolyser par les enzymes protéolytiques du pancréas et donc par l'obtention d'un meilleur rapport enzyme/substrat. De plus, avec la matière protéique à vitesse de digestion lente il existe un meilleur gain protéique post-prandial.

Dans les maladies où il existe des dysfonctionnements du métabolisme des acides aminés et plus spécifiquement des déficits enzymatiques dans la voie de dégradation de ces acides aminés (phénylalaninémie et phenylcétonurie, hypertyrosinémie, histidinémie, homocystinurie, aminoacidopathies liées aux acides aminés branchés, par exemple), l'accumulation de ces acides aminés ou d'un de leur produit de dégradation génère des complications neurologiques et cliniques. Afin d'éviter cette accumulation, un traitement diététique est prescrit. Il consiste en un régime qui ne contient pas - ou en très faible quantité - l'acide aminé incriminé dans le développement de la maladie. Les produits spécifiques développés pour ces populations sont composés soit d'acides aminés libres, soit de protéines fortement hydrolysées. Cependant, ces mélanges ne possèdent pas un goût agréable. De plus, afin d'éviter les diarrhées consécutives à l'hyperosmolarité des produits, les consommateurs doivent ingérer les produits sous forme de petits repas. La matière protéique qui possède à la fois une vitesse de digestion lente et un faible contenu en l'acide aminé incriminé, permet d'améliorer le goût et donc la tolérance du régime, de limiter les risques de diarrhée, d'éviter les fluctuations plasmatiques en acides aminés, et de d'augmenter le gain protéique postprandial.

L'utilisation de matière protéique à vitesse de digestion lente peut aussi être envisagée pour des personnes qui ne présentent pas de dénutrition, comme les prématurés, les nouveau-nés, les enfants, les obèses et les personnes âgées, par exemple.

L'ingestion de matière protéique à vitesse de digestion lente, chez les prématurés, les nouveau-nés ou les enfants non dénutris, en assurant un meilleur rendement d'utilisation des protéines alimentaires est susceptible de favoriser la croissance de l'organisme.

La matière protéique à vitesse de digestion ralentie, en diminuant la prise alimentaire par un mécanisme satiétogène peut être administrée aux personnes présentant des troubles de l'homéostasie pondérale (obésité) ou lors d'épisodes de boulimies. Elle peut limiter la diminution de la masse protéique consécutive au suivi d'un régime alimentaire hypocalorique. Ces deux effets conjugués permettent de diminuer leur masse grasse avec d'une part une plus grande facilité pour réduire leurs apports et d'autre part une meilleure conservation de leur masse protéique.

Chez les personnes âgées, par rapport aux sujets jeunes il existe une diminution de la masse protéique corporelle, diminution qui présente une incidence sur l'autonomie, la résistance aux agressions (maladies, stress divers) et la faculté de récupération à ces agressions. De plus le vieillissement est associé à une diminution de l'activité rénale. La matière protéique à vitesse de digestion lente en permettant donc une meilleure conservation de la masse protéique permet ainsi d'éviter les surcharges rénales.

La matière protéique à vitesse de digestion ralentie, en apportant les acides aminés d'une manière plus continue et régulière, permet de favoriser la synthèse de nouveaux matériaux tissulaires intervenant dans les processus de cicatrisation ou de régénération des tissus biologiques.

La matière protéique à vitesse de digestion ralentie peut être destinée à la nutrition des animaux familiers, en particulier celle des sujets âgés et des jeunes en période de croissance. Elle peut également être administrée à certains sujets de manière à contrôler leur poids corporel.

Les compositions selon l'invention peuvent contenir une source de protéines fournissant au moins 8% de l'énergie totale, une source de glucide fournissant jusqu'à 70% de l'énergie totale et une source de lipides fournissant jusqu'à 35% de l'énergie totale.

Les protéines contenues dans les compositions selon l'invention peuvent fournir de 5 à 100% de l'énergie totale, en particulier de 8 à 30% et de préférence de 10 à 20%. Dans le cas des compositions destinées à l'alimentation des animaux de compagnie, la teneur en protéines peut atteindre 40% sur la base de l'extrait sec.

Ces compositions comprennent, de préférence, une source de glucides fournissant 0 à 70% de l'énergie totale. Les glucides, sont des nutriments importants pour rétablir le bilan énergétique. Tous les glucides peuvent être utilisés, notamment les maltodextrines, le saccharose, le lactose et le.glucose, par exemple.

Les compositions peuvent comprendre une source en lipides fournissant jusqu'à 35% de l'énergie totale. Les huiles végétales sont recommandées, notamment provenant celles du soja, du palmier à huile, du cocotier, du tournesol, etc. Dans le cas des compositions destinées à l'alimentation des animaux de compagnie, la source de lipides peut fournir jusqu'à 60% de l'énergie totale.

La valeur énergétique de ces compositions peut être comprise entre 70 et 200 Kcal/ 100ml, par exemple.

Dans le cas des compositions destinées à la nutrition infantile, les protéines représentent de préférence 0,45 à 0,7 g/100 kJ, les carbohydrates de préférence 1,7-3,4 g/ 100 kJ et les lipides de préférence 0,8-1,5 g/100 kJ.

Dans le cas de compositions destinées à des patients atteints de phénylcétonurie, la matière protéique peut contenir environ 50% de caséino-glyco-macropeptides, une source de carbohydrates, une source lipidique et des vitamines et minéraux.

Les compositions selon la présente invention peuvent être préparées de toutes sortes de manières, les étapes de fabrication incluant généralement une dispersion des ingrédients dans de l'eau, une émulsification et une pasteurisation.

Les compositions peuvent être préparées sous forme de boissons ou de concentrés liquides ou semi-solides ou sous forme d'une poudre que l'on peut reconstituer dans de l'eau, par exemple. Elles peuvent également se présenter sous une forme solide telle que céréales, barres nutritionnelles par exemple.

Des minéraux, des vitamines, des sels, des émulsifiants ou des composés aromatiques peuvent aussi être ajoutés aux compositions, selon les besoins. Les vitamines et minéraux pouvant représenter de 25 à 250% des apports journaliers recommandés. Dans le cas de formules infantiles, on ajoute les quantités de vitamines et minéraux prescrites par la Directive Européenne.

La présente invention est décrite plus en détail ci-après à l'aide des exemples, donnés à titre d'illustration de l'objet de l'invention dont ils ne constituent en aucune manière une limitation. Les pourcentages sont donnés en poids sauf indication contraire. Ces exemples sont précédés d'une brève description des figures.
**Figure 1:** présente le pourcentage de protéines hydrolysées en fonction du temps pour la digestion *in vitro* de lactosérum natif, lactosérum natif + alginate et lactosérum microparticulé + alginate.
**Figure 2:** représente le pourcentage d'azote ingéré et demeurant dans le tube digestif (estomac + intestin grêle) en fonction du temps lors de la digestion *in vivo* de solutions de lactosérum natif, lactosérum natif + alginate ou lactosérum microparticulé + alginate.
**Figure 3 :** représente le pourcentage d'azote ingéré et demeurant dans le tube digestif (estomac + intestin grêle) en fonction du temps lors de la digestion *in vivo* de repas complets contenant des protéines de lactosérum natif (■) ou du lactosérum modifié + alginate (●).
**Figure 4:** représente les courbes de "faim", "envie de manger" et "distension de l'estomac" pour des repas à base de protéines de lactosérum natif (NW) et modifié (MW) en fonction du temps.

### Exemple 1 : Cinétique de digestion des solutions protéiques

Les microparticules de lactosérum sont préparées à partir d'un mélange de solution d'alginate (Manucol DM, Kelco) à 3% à pH 7 et d'une solution de protéines sériques à 15% (Lacprodan DI-9223; Danmark Protein) dont le pH est de 6,6.

Les concentrations dans le mélange sont de 1% d'alginate et 10% de protéines. Le mélange est chauffé à 80°C pendant 10 minutes et sera dilué deux fois pour obtenir une concentration finale en protéines de 5 %. Les microparticules ont un diamètre entre 500 nm et 5 microns.

L'hydrolyse enzymatique in vitro et in vivo des microparticules est comparée à celle d'une solution de protéines natives à 5% et à celle d'un mélange de protéines natives (5%) et d'alginate (0,5%).

La digestion enzymatique in vitro est effectuée d'après la méthode décrite par Savalle et al. (*J. Agric*. *Food Chem*., **37**, 1336, 1989) et adaptée de la manière suivante : les échantillons, contenant 250 mg de protéines, sont incubés à 37°C en présence de pepsine (1 mg) à pH 1.9 pendant 30 minutes. Le milieu est ensuite neutralisé à pH 7,5 avec de la soude et une digestion à la pancréatine pendant 5h30 est effectuée. Le degré d'hydrolyse est déterminé par la mesure des groupes aminés libres par la méthode au TNBS (Adler-Nissen, *J. Agric. Food Chem*, **27,** 1256, 1979). Avant l'incubation, les échantillons sont broyés par passage dans une seringue de 1 mm de diamètre afin de simuler in vitro les conditions de gavage qui sont employées in vivo chez le rat.

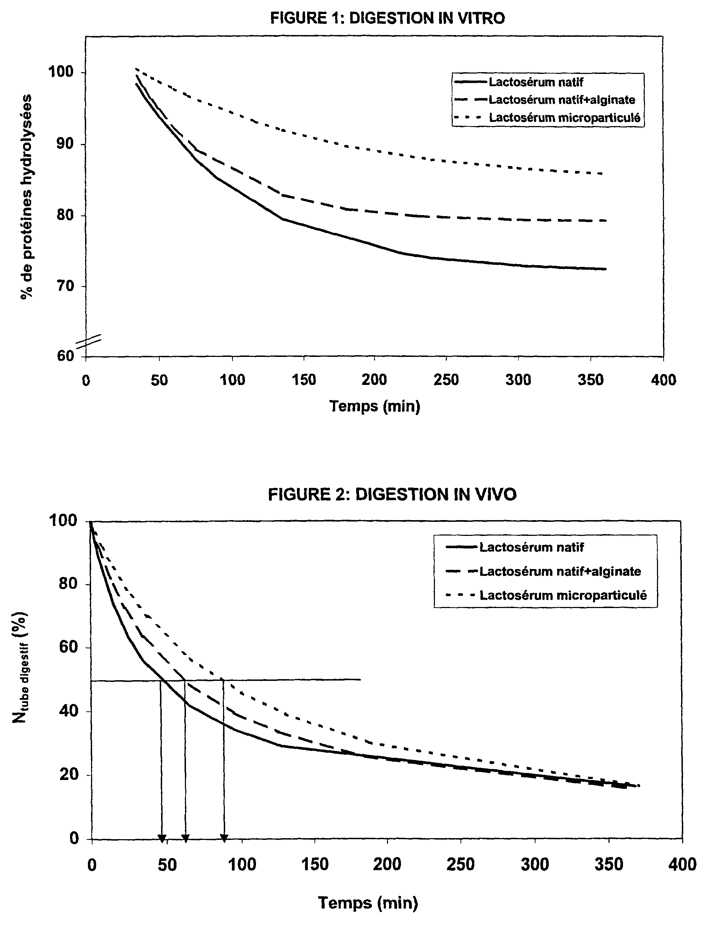

La cinétique d'hydrolyse in vitro est représentée sur la figure 1. Les résultats montrent que le lactosérum microparticulé est digéré plus lentement que le lactosérum natif contenant ou non de l'alginate, et ce plus particulièrement pendant les deux premières heures d'hydrolyse.

In vitro, le lactosérum natif n'est hydrolysé qu'à 30 % environ (la valeur 100 % de protéines non hydrolysées reportée sur le graphe correspond à la quantité de groupes NH2 contenus dans le lactosérum, selon Adler-Nissen).

Pour l'étude de la digestion in vivo, 21 rats mâles Sprague-Dawley (Iffa-Credo, F-6210 L'Arbresle, France), pesant de 160 à 180 g sont répartis de manière aléatoire en 11 lots. Après une période d'acclimatation d'au moins 2 jours, les animaux sont placés dans des cages métaboliques (pour éviter la coprophagie) et soumis à un jeûne de 22 heures. Puis, les rats sont nourris par gavage d'une suspension de 5ml de protéine à tester à 5%.

Les rats sont anesthésiés à 0, 10, 20, 30, 60, 90, 120, 180, 240, 360 minutes à partir du gavage. La cavité abdominale est ouverte et des échantillons de sang sont prélevés de la veine porte et de l'aorte dorsale. Les animaux sont ensuite sacrifiés ; l'estomac et le petit intestin sont séparés de la cavité abdominale. Les contenus gastrique et intestinal sont récupérés par lavage du contenu luminal avec une solution de NaCl à 0,9%.

Les échantillons de sang sont mélangés à de l'héparine et centrifugés. Les échantillons de plasma sont déprotéinés avec de l'acide sulfosalycilique à 3,6% (w/v, concentration finale) puis conservés à - 80°C jusqu'à l'analyse des acides aminés (amino acid analyser, System 6300-Beckman).

Les contenus gastro-intestinaux sont maintenus au froid et leur teneur en azote total est rapidement analysé.

Le pourcentage d'azote ingéré et demeurant dans le tube digestif (estomac + intestin grêle) en fonction du temps est représentée sur la figure 2.

Les résultats montrent qu'en solution, le lactosérum microparticulé est digéré moins vite que le lactosérum natif, et que cet effet est du à la modification et non à la présence d'alginate. La disparition de la moitié de l'azote ingéré du tube digestif a lieu après 90 min pour le lactosérum microparticulé alors qu'elle a lieu après 45 minutes pour le lactosérum natif.

### Exemple 2 : cinétique de digestion des protéines contenues dans des repas complets.

On procède tel que décrit à l'exemple 1, à la différence que les rats sont gavés avec un repas complet de composition suivante (% en poids) : 5% de protéines de lactosérum natif ou microparticulé, 8% d'huile de soja, 0,1% d'émulsifiant, 17% de saccharose, 8% de maltodextrines et 61,9% d'eau.

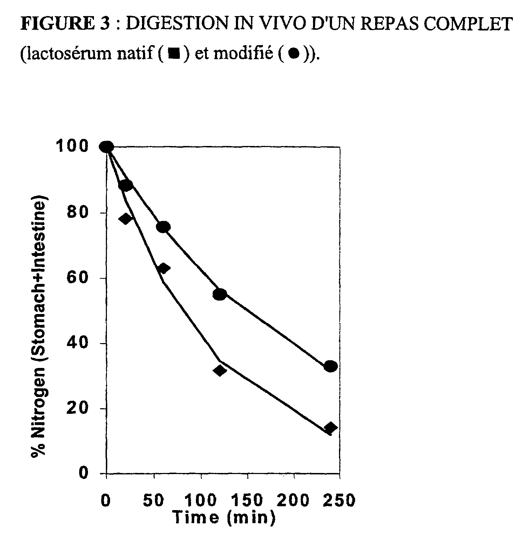

Les résultats présentés dans la figure 3 indiquent qu'au sein d'un repas complet, la protéine dont la vitesse de digestion a été ralentie est plus lentement digérée que la protéine native. La disparition de la moitié de l'azote ingéré du tube digestif a lieu après 145 minutes pour le lactosérum microparticulé alors qu'elle a lieu après 78 minutes pour le lactosérum natif.

### Exemple 3 : Etude de la satiété sur volontaires humains

### Matériel et méthodes

### Echantillons:

L'isolat de protéines de lactosérum **(NW)** a été fourni par MD-Foods. Les solutions protéiques à boire ont été préparées en mélangeant dans de l'eau déminéralisée les ingrédients donnés dans la Table 1, puis laissées pendant la nuit à 4 °C sous agitation.

Les protéines de lactosérum microparticulées **(MW)** ont été préparées à partir du **NW** selon les étapes suivantes:
1) dissoudre l'alginate et le reste des ingrédients séparément dans l'eau.
2) mélanger les 2 solutions de manière à obtenir la composition finale donnée dans la Table 1, et répartir la composition dans des boîtes en acier inoxydable de 200g chacune, scellées puis chauffées dans un four à une température de 105 °C jusqu'à ce que la température interne atteigne 78 °C, puis refroidies.
3) Les boissons et le gel de protéines de lactosérum sont aromatisées, sucrées et colorées pour améliorer leur palatabilité. Des arômes de différentes sortes et à des concentrations différentes ont été testés par un panel de 8 personnes et les produits et doses les plus fréquemment choisis ont été utilisés pour les compositions finales (Table 1).

Les repas (400 g) sont isoenergétiques (178 Kcal/portion) et isoprotéiniques (40 g/portion).

**Table 1**

| Composition des repas protéiniques ( en g par ration de 400 g) | | |
|---|---|---|
| | **NW** | **MW** |
| Isolat de protéines de lactosérum | 47,6 | 47,6 |
| Alginate de sodium | | 2,0 |
| Edulcorant artificiel | 1,6 | 1,6 |
| Saccharose | 8,0 | 8,0 |
| Arôme caramel | 0,8 | 0,8 |
| Colorant caramel | 0,02 | 0,02 |
| eau | 342,0 | 340,0 |

### Sujets

5 volontaires humains d'âge moyen 32,5 ± 6,9 et d'index de masse corporelle moyenne de 22,3 ± 1,7 Kg/m², ont reçu un des 2 repas à chacun des 2 jours de l'expérience.

### Protocole

Les sujets n'ont pas consommé de boissons alcoolisées la veille de l'étude et ont pris un dîner léger avant 20h00 et jeûné jusqu'au début du protocole. 3 repas ont été consommés le jour du protocole:
1) un petit-déjeuner léger et standardisé, consistant en une tranche de pain entier, 5 g de beurre, 10 g de confiture et du café ou du thé avec du lait (150 Kcal). Il a été servit à 7h45 et a été consommé en 10-15 min.
2) les repas tests ont été servis à 10h00 et consommés en 15 minutes environ.
3) Un repas à base de pâtes avec sauce tomate et kiwis a été servi à 13h00.

Les volontaires ont noté leur sentiments de faim, envie de manger et de distension de l'estomac, sur une échelle analogique visuelle (10 cm) à des intervalles de 30 minutes entre 10h00 et 13h00.

Lors du repas de 13h00, où les volontaires ont mangé à leur faim, la quantité de pates et sauce tomate ingérée a été contrôlée par pesée. La quantité de kiwis consommée a été contrôlée par unité (100 ± 5 g par unité). Les sujets ont noté sur un carnet les aliments consommés le reste de la journée d'étude. La quantité des différents aliments consommés lors du repas de midi et lereste de la journée a permis d'estimer le nombre de Kcal ingérés, en utilisant la Table de composition d'aliments de McCance et Widdowson (1991).

### Résultats

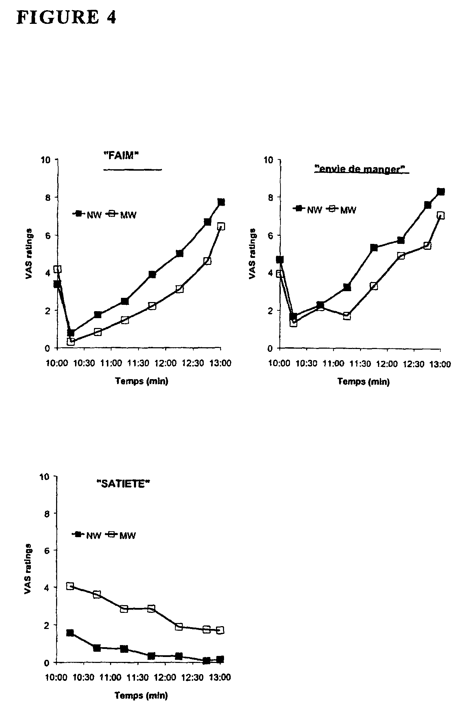

Les courbes de "faim", "envie de manger" et "distension de l'estomac" sont données figure 4. Les protéines de lactosérum natif (NW) et modifié (MW) se comportent différemment. Le retour de la faim et du désir de manger est plus lent avec le repas à base de MW et le sentiment de distension de l'estomac perdure plus longtemps pour le MW.

On a comparé l'apport calorique moyen au repas et pendant le reste de la journée après une première charge de NW et MW. Les résultats table 2 montrent que dans le cas de MW, cet apport est réduit.

Les résultats montrent un effet plus rassasiant du lactosérum modifié par rapport au lactosérum natif.

**Table 2:**

| apport calorique en Kcal | | |
|---|---|---|
| | NW | MW |
| Repas | 1459 ± 772 | 1091 ± 333 |
| Reste de la journée | 933 ± 273 | 731 ± 262 |
| repas + reste de la journée | 2392 ± 682 | 1822 ± 547 |

### Exemple 4: Etude de la qualité nutritionnelle de la protéine modifiée

### Matériel et méthodes

Les protéines de lactosérum microparticulées MW, ont été préparées en mélangeant une solution d'alginate à 2% en poids et 20% en poids d'une solution de protéines de lactosérum dans un rapport 1:1. La composition est répartie dans des boîtes de 200 ml puis traitées tel que décrit dans l'exemple 2.

Deux diètes sont préparées en mélangeant dans un mixeur les ingrédients présentés table 3. Elles sont fournis pendant 21 jours à 2 lots de 10 rats mâles Sprague Dawley pesants environ 60g au début de l'étude. On mesure l'évolution pondérale, ainsi que la quantité de régime ingérée pendant les 3 semaines. La deuxième semaine d'étude les animaux sont transférés dans - des cages métaboliques et on procède à la récolte des selles et d'urine pendant 7 jours.

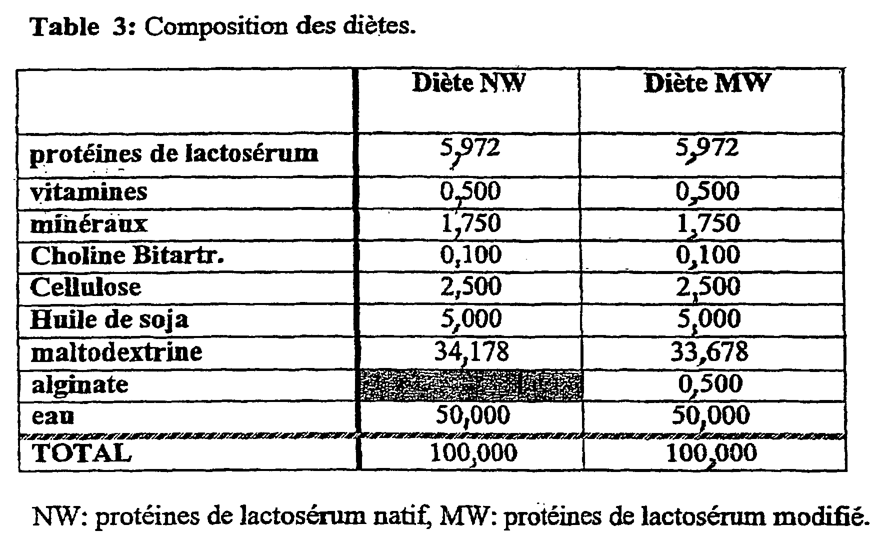

On a ensuite mesuré les paramètre suivants:
- la digestibilité (D),
- la valeur biologique (BV),
- l'utilisation protéique nette (NPU)
- le rapport d'efficacité protéique (PER)

Les résultats donnés tables 4 montrent une légère diminution de la digestibilité de l'azote dans le cas d'une diète MW, qui n'a pas d'effet sur l'utilisation nette de la protéine (NPU) grâce à une utilisation de l'azote absorbé (BV) légèrement meilleure. Le rapport d'efficacité protéique n'est d'ailleurs pas affecté par le traitement (PER).
Les résultats montrent que la microparticulation de la protéine n'altère pas sa qualité nutritionnelle.

**Table 4:**

| PER; digestibilité, BV and NPU de rats nourris avec diète NW et diète MW pendant 21 jours (moyenne ± 95% intervalle de confiance). | | | | |
|---|---|---|---|---|
| | **PER** | **Digestibilité** | **BV** | **NPU** |
| NW | 3,38 ± 0,11 | 97,6 ± 0,3 | 68,7 ± 6,7 | 67,0 ± 4,7 |
| MW | 3,30 ± 0,18 | 95,6 ± 0,4 (*) | 73,1 ± 4,1 | 69,9 ± 2,8 |

| | | | | |
|---|---|---|---|---|
| (*) Significativement différent à p < 0,05 | | | | |

### Exemple 5: Composition alimentaire pour nourrissons

On prépare une composition alimentaire pour nourrissons sous forme d'une poudre soluble ayant la composition définie dans la table 5 ci-dessous. Cette poudre est utilisée à raison de 13% dans de l'eau, ce qui correspond à une densité énergétique de l'ordre de 70 kcal/100 ml.

Pour préparer cette poudre, on purifie de l'eau par osmose inverse, on la chauffe à 70°C, on lui ajoute une source de protéines et une source de carbohydrates, on lui ajoute une source de lipides dans laquelle on a préalablement dispersé les vitamines lipoposolubles, on chauffe le mélange à 80°C pendant 5 min par injection de vapeur, on le refroidit à 60°C, on lui ajoute les minéraux et les vitamines hydrosolubles, on l'homogénéise en 2 étapes à 10 mPa puis à 7 mPa, on le sèche par pulvérisation sous un flux d'air chaud jusqu'à une teneur en eau de 4%, puis on le réduit en une fine poudre soluble dans l'eau.

On ajoute à la composition les vitamines et minéraux en quantité satisfaisant les apports journaliers recommandés.

**Table 5**

| | | |
|---|---|---|
| PROTEINES | | 2,3 g/100 Kcal |
| | Caséine | 40 % |
| | Lactosérum traité selon l'exemple 1 | 60% |
| GLUCIDES | | 10 g/100 Kcal |
| | Lactose | 100 % |
| LIPIDES | | 5,5 g/100 Kcal |
| | Graisse de lait | 70% |
| | Huile de canola | 15% |
| | Huile de maïs | 14 % |
| | Lécithine de soja | 1 % |

### Exemple 6 : Composition entérale

On prépare une composition entérale liquide contenant les ingrédients définis dans la table 6 ci-dessous, de la même manière qu'à l'exemple 5 à la différence près qu'on homogénéise le mélange à 150°C par injection de vapeur, on le refroidit à 75°C et on le conditionne asseptiquement dans des récipients. On ajoute les vitamines et minéraux en quantité satisfaisant les apports journaliers recommandés.

Cette composition présente une densité énergétique de 100 Kcal/100 ml.

**Table 6**

| | | |
|---|---|---|
| PROTEINES | | 6,5 g/100 ml |
| | Lactosérum traité selon l'exemple 1 | 100 % |
| GLUCIDES | | 11,3 g/100 ml |
| | Solides d'un sirop de mais | 56 % |
| | Saccharose | 34,4 % |
| | Xanthane | 9,6% |
| LIPIDES | | 3,4 g/100 ml |
| | Huile de noix de coco | 50 % |
| | Huile de canola | 30 % |
| | Huile de maïs | 14 % |
| | Lécithine de soja | 6 % |

### Exemple 7 : Supplément nutritionnel pour des malades souffrant d'insuffisance rénale.

On prépare une composition liquide destinée à des personnes qui souffrent d'insuffisance rénale, contenant les ingrédients définis dans la table 7 ci-dessous, de la même manière qu'à l'exemple 6. On ajoute les vitamines et minéraux en quantité satisfaisant les apports journaliers recommandés.

Cette composition présente une densité énergétique de 200 Kcal/100 ml.

**Table 7**

| | | |
|---|---|---|
| PROTEINES | | 5 g/100 ml |
| | Lactosérum traité selon l'exemple 1 | 100 % |
| GLUCIDES | | 27 g/100 ml |
| | Solides d'un sirop de mais | 56 % |
| | Maltodextrine | 34,4 % |
| | Saccharose | 9,6 % |
| LIPIDES | | 8 g/100 ml |
| | Huile de noix de coco | 50 % |
| | Huile de canola | 30 % |
| | Huile de maïs | 14 % |
| | Lécithine de soja | 6 % |

### Exemple 8 : Composition alimentaire pour malades souffrant de phénylcétonurie

On prépare une composition alimentaire pour phénylcetonuriques de la même manière qu'à l'exemple 5, sous forme d'une poudre soluble et ayant la composition définie dans la table 8 ci-dessous. On ajoute les vitamines et minéraux en quantité satisfaisant les apports journaliers recommandés.

Cette poudre est utilisée à raison de 15% dans de l'eau, ce qui correspond à une densité énergétique de l'ordre de 70 kcal/100 ml et à un contenu en phénylalanine de l'ordre de 10 mg/100 ml.

**Table 8**

| | | |
|---|---|---|
| PROTEINES | | 3,3 g/100 Kcal |
| | Caséino-glycomacropeptide traité selon l'exemple 1 | 50 % |
| | Acides aminés libres | 50% |
| L-Arginine, L-Cystine, L-Glutamine, L-Glycine, L-Histidine, L-Isoleucine, L-Leucine, L-Lysine, L-Methionine, L-Proline, L-Tryptophan, L-Tyrosine, L-Valine. | | |
| GLUCIDES | | 13 g/100 Kcal |
| | Lactose | 100 % |
| LIPIDES | | 3,9 g/100 Kcal |
| | Huile de canola | 60 % |
| | Huile de maïs | 39 % |
| | Lécithine de soja | 1 % |

### Exemple 9 : Supplément nutritionnel hypocalorique.

On prépare une composition nutritionnelle destinée à des personnes qui désirent réduire ou maintenir leur poids, sous forme d'une poudre soluble, aromatisée au chocolat, et ayant la composition définie dans la table 9 ci-dessous. On ajoute les vitamines et minéraux en quantité satisfaisant les apports journaliers recommandés.

Cette poudre est utilisée à raison de 13% dans du lait écrémé, ce qui correspond à une densité énergétique de l'ordre de 100 kcal/100 ml.
Pour préparer cette poudre, on mélange tous les ingrédients à sec, on conditionne par humidification et re-séchage jusqu'à une teneur en eau de 4%, puis on réduit le mélange en une fine poudre soluble dans l'eau.

**Table 9**

| | | |
|---|---|---|
| PROTEINES | | 35 g/100 g |
| Lactosérum **traité selon l'exemple 1** | | 100 % |
| GLUCIDES | | 63 g/100 g |
| | Saccharose | 65 % |
| | Maltodextrine | 10 % |
| | Cellulose | 25 % |

### Exemple 10 : Composition aromatisée pour personnes âgées.

On prépare comme décrit à l'exemple 6, une composition nutritionnelle liquide, destinée à des personnes âgées, aromatisée à la fraise et ayant la composition définie dans la table 10 ci-dessous. On ajoute les vitamines et minéraux en quantité satisfaisant les apports journaliers recommandés.

**Table 11**

| ***INGREDIENTS*** | ***COMPOSITION (g*/*100g)*** |
|---|---|
| Sucrose | 6,0750 |
| Maltodextrines | 4,8830 |
| Protéines | 7,5000 |
| Huile de colza | 1,3550 |
| Huile de mais | 0,4670 |
| Amidon de mais | 0,4070 |
| Arôme fraise | 0,1960 |
| Monoglycérides | 0,1830 |
| Vitamine C | 0,0324 |
| phosphate de Na dibasique | 0,0249 |
| Carageenanes iota | 0,0247 |
| Micronutriments | 0,0187 |
| Choline chloride | 0,0183 |
| Minéraux : K, Fe, Zn, Mg | 0,2704 |
| Colorant | 0,0020 |
| Eau | 78,566 |
| **TOTAL** | **100,0000** |
| **Calories/g** | **1,00** |

### Exemple 11 composition pour l'alimenation des animaux de compagnie

On prépare trois variables d'un aliment hautement palatable pour chats à base de viandes auxquelles on rajoute des premix de minéraux et de vitamines, ainsi que de la taurine. L'ensemble est gélifié soit par ajout de gomme guar à 0,3% (variable A), soit par ajout de gomme xanthane à 0,5 % (variable B). La gomme guar et la gomme xanthane sont ajoutées après hydratation. Cependant, la variable B contenant la gomme xanthane est ensuite broyée finement au moyen d'un appareil rotatif à grille. La troisième variable (variable C) est identique à la variable A, mais elle est traitée par broyage fin de la même façon que la variable B contenant la gomme xanthane. Les variables sont ensuite conditionnées dans de boîtes de contenance 156g puis stérilisées dans des autoclaves industriels. Bien que l'appétance reste similaire entre les 3 variables, la gomme xanthane contribue à une texture nettement différente de celle des autre variables.

La composition nutritionnelle des variables est indiquée ci-dessous:

| | Humidité (%) | Protéines (g/100g) | Graisse (g/100g) | Fibres (%) | Cendres (%) | Carbohydrates (%) |
|---|---|---|---|---|---|---|
| Variable A | 79,4 | 12,9 | 5,2 | 0,14 | 1,65 | 0,65 |
| Variable B | 79,8 | 12,5 | 5,3 | 0,10 | 1,41 | 0,82 |
| Variable C | 79,4 | 12,8 | 5,1 | 0,11 | 1,88 | 0,70 |

Un groupe de 36 chats adultes a consommé un aliment similaire à la diète contrôle pendant une semaine, puis a été séparé en 3 groupes de 12 chats chacun consommant soit la variable A, soit la variable B, soit la variable C, pendant 13 jours. Au bout des 13 jours, les traitements ont été alternés pour encore 13 jours. Ainsi, chaque chat a reçu deux variables, chacune pendant 13 jours, selon un plan d'expérience en crossover à bloc ouvert.

Au cours du test il a été observé que certains chats avaient des selles molles: Il en a donc été tenu compte dans l'interprétation des résultats.

Lors de la première phase de l'étude, il a été observé que les chats recevant la variable B perdaient plus de poids de façon statistiquement significative à p= 0,05 que pour les autres variables, malgré des prises alimentaires similaires. Cet effet était maintenu lorsque les résultats des chats ayant eu des selles molles ont été exclus de l'analyse, la différence de perte de poids entre les variables A et B restant statistiquement significative:

| | Prise alimentaire (g/jour.chat) | Evolution du poids (% du poids initial) Tous chats selles molles exclues | |
|---|---|---|---|
| Variable A | 41,1 +- 11,9 | -0,11 +- 2,76 | +0,03 +- 2,85 |
| Variable B | 41,7 +- 12,7 | -4,01 +- 2,52 | -3,22 +- 1,78 |
| Variable C | 43,4 +- 11,2 | -1,81 +- 2,56 | -1,88 +- 2,75 |

Ce résultat montre bien l'intérêt de la présente invention pour le contrôle du poids corporel chez les animaux de companie.

### Exemple 12 : Aliment pour chiots

Un aliment extradé complet pour chiots est préparé à base de céréales et de sources de protéines. Sa composition nutritionnelle est la suivante: protéines au moins 22%, lipides au moins 8%, fibres 4,5% environ, humidité 12% au plus, calcium au moins 1%, phosphore au moins 0,8%. L'ajout de gomme xanthane dans la composition par des moyens appropriés permet d'obtenir des effets bénéfiques sur la croissance des chiots.

### Exemple 13

Aliment extrudé tel que décrit à l'exemple 12, dans lequel la teneur en lipides d'au moins 5% mais inférieure à 8%. L'ajout de gomme xanthane dans la composition par des moyens appropriés permet d'aider à limiter la prise de poids corporel des chiens.

## Revendications

1. Utilisation d'une matière protéique à digestion lente pour la préparation d'une composition destinée à être administrée par voie entérale à un mammifère afin de moduler le taux plasmatique post-prandial en acides aminés, ladite matière protéique ayant été préalablement traitée de sorte à transformer les protéines à digestion rapide qu'elle contenait en protéines à digestion lente, **caractérisée en ce que** la matière protéique à digestion lente est une matière contenant des protéines gélifiées microparticulées associées à des polysaccharides dans des conditions d'incompatibilité thermodynamique.

2. Utilisation d'une matière protéique selon la revendication 1, pour la préparation d'une composition destinée à:
a) accroître le gain protéique post-prandial, et/ou
b) éviter une surcharge métabolique de certains organes et/ou de certaines enzymes et/ou
c) limiter la prise alimentaire quotidienne grâce à un effet satiétogène de ces protéines et/ou
d) pallier à certains dysfonctionnements du métabolisme des acides aminés.
e) d'améliorer la régénération des tissus, notamment les processus de cicatrisation.

3. Utilisation d'une matière protéique selon la revendication 1, pour la préparation d'une composition destinée à la nutrition des patients atteints d'insuffisance rénale et ou de pathologies hépatiques.

4. Utilisation d'une matière protéique selon la revendication 1, pour la préparation d'une composition destinée à la nutrition des patients atteints de dysfonctionnements du métabolisme des acides aminés ou plus spécifiquement de déficits enzymatiques.

5. Utilisation d'une matière protéique selon la revendication 1, pour la préparation d'une composition destinée à l'alimentation des animaux de compagnie, notamment les sujets âgés, les jeunes sujets en période de croissance et certains sujets pour contrôler leur poids corporel.

6. Utilisation d'une matière protéique selon la revendication 1, **caractérisée en ce que** les protéines sont gélifiées par traitement thermique.

7. Utilisation d'une matière protéique selon la revendication 1, **caractérisée en ce que** les polysaccharides sont choisis parmi les alginates, la gomme xanthane, la gomme arabique, le guar, l'amidon, les maltodextrines, les dextrines, les pectines, les kappa-carraghénanes, les iota-carraghénanes, les lambda-carraghénanes, la méthyl-cellulose et la carboxy-méthyl-cellulose, les dextranes sulphatés et/ou la gomme gellane.

8. Composition destinée à être administrée par voie entérale à un mammifère, contenant une matière protéique à digestion lente, ladite matière protéique ayant été préalablement traitée de sorte à transformer les protéines à digestion rapide qu'elle contenait en protéines à digestion lente, **caractérisée en ce que** la matière protéique à digestion lente est une matière contenant des protéines gélifiées microparticulées associées à des polysaccharides dans des conditions d'incompatibilité thermodynamique.

9. Composition selon la revendication 8, **caractérisée en ce que** les polysaccharides sont choisis parmi les alginates, la gomme xanthane, la gomme arabique, le guar, l'amidon, les maltodextrines, les dextrines, les pectines, les kappa-carraghénanes, les iota-carraghénanes, les lambda-carraghénanes, la méthyl-cellulose et la carboxy-méthyl-cellulose, les dextranes sulphatés et/ou la gomme gellane.

10. Composition selon l'une des revendications 8 ou 9, destinée à:
a) accroître le gain protéique post-prandial, et/ou
b) éviter une surcharge métabolique de certains organes et/ou de certaines enzymes et/ou
c) limiter la prise alimentaire quotidienne grâce à un effet satiétogène de ces protéines et/ou
d) pallier à certains dysfonctionnements du métabolisme des acides aminés.
e) d'améliorer la régénération des tissus, notamment les processus de cicatrisation

11. Composition selon l'une des revendications 8 à 10, contenant une source de protéines fournissant au moins 8% de l'énergie totale, une source de glucides fournissant jusqu'à 70% de l'énergie totale et une source de lipides fournissant jusqu'à 35% de l'énergie totale.

12. Composition selon l'une des revendications 8 à 11, ayant une densité énergétique comprise entre 70 et 200 Kcal/ 100ml.

13. Composition selon l'une des revendications 8 à 12 destinée à des patients atteints de phénylcétonurie, dans laquelle la matière protéique contient environ 50% de caséino-glyco-macropeptides, une source de carbohydrates, une source lipidique et des vitamines et minéraux.

14. Composition selon l'une des revendications 8 à 13 destinée à l'alimentation des animaux familiers.

15. Composition selon la revendication 14 destinée à l'alimentation des sujets âgés, des jeunes sujets en période de croissance et de certains sujets pour contrôler leur poids corporel.

16. Composition selon la revendication 14, dans laquelle la source de lipides représente jusqu'à 60% de l'énergie totale et la quantité de protéines jusqu'à 40% sur base de l'extrait sec.

## Claims

1. Use of a slowly digestible protein material for the preparation of a composition designed for enteral administration to a mammal so as to modulate the postprandial level of amino acids in the plasma, the said protein material having been previously treated so as to convert the rapidly digestible proteins which it contains into slowly digestible proteins, **characterized in that** the slowly digestible protein material is a material containing microparticulate gelled proteins associated with polysaccharides under conditions of thermodynamic incompatibility.

2. Use of a protein material according to claim 1, for the preparation of a composition designed to:
a) increase the postprandial gain in proteins, and/or
b) prevent a metabolic overload of certain organs and/or of certain enzymes and/or
c) limit the daily nutritional intake of these proteins by means of an effect giving a sensation of satiety and/or
d) to make up for certain malfunctions of the metabolism of amino acids,
e) to improve the regeneration of tissues, in particular scarring processes.

3. Use of a protein material according to claim 1, for the preparation of a composition designed for the nutrition of patients suffering from renal failure or with hepatic pathological conditions.

4. Use of a protein material according to claim 1, for the preparation of a composition designed for the nutrition of patients suffering from malfunctions of the metabolism of amino acids or more specifically from enzyme deficiencies.

5. Use of a protein material according to claim 1, for the preparation of a composition designed for the nutrition of pets, particularly elderly subjects, of young subjects during a period of growth and of certain subjects to control their body weight.

6. Use of a protein material according to claim 1, **characterized in that** the proteins are gelled by heat treatment.

7. Use of a protein material according to claim 1, **characterized in that** the polysaccharides are chosen from alginates, xanthan gum, gum arabic, guar gum, starch, maltodextrins, dextrins, pectins, kappa-carrageenans, iota-carrageenans, lambda carrageenans, methylcellulose and carboxymethylcellulose, sulfated dextrans and/or gellan gum.

8. Composition designed for enteral administration to a mammal, containing a slowly digestible protein material, the said protein material having been previously treated so as to convert the rapidly digestible proteins that it contains into slowly digestible proteins, **characterized in that** the slowly digestible protein material is a material containing microparticulate gelled proteins associated with polysaccharides under conditions of thermodynamic incompatibility.

9. Composition according to claim 8, **characterized in that** the polysaccharides are chosen from alginates, xanthan gum, gum arabic, guar gum, starch, maltodextrins, dextrins, pectins, kappa-carrageenans, iota-carrageenans, lambda carragheenans, methylcellulose and carboxymethylcellulose, sulfated dextrans and/or gellan gum.

10. Composition according to either of claims 8 or 9, designed to:
a) increase the postprandial gain in proteins, and/or
b) prevent a metabolic overload of these organs and/or of certain enzymes and/or
c) limit the daily nutritional uptake of certain proteins by means of an effect giving a sensation of satiety and/or
d) to make up for certain malfunctions of the metabolism of amino acids,
e) to improve the regeneration of tissues, in particular scarring processes.

11. Composition according to one of claims 8 to 10, containing a source of proteins providing at least 8 % of the total energy, a source of carbohydrates providing up to 70 % of the total energy and a source of lipids providing up to 35 % of the total energy.

12. Composition according to one of claims 8 to 11, having an energy density of between 70 and 200 Kcal/100 ml.

13. Composition according to one of claims 8 to 12, designed for patients suffering from phenylketonuria, wherein the protein material contains approximately 50 % caseino-glyco-macropeptides, a source of carbohydrates, a source of lipids and vitamins and minerals.

14. Composition according to one of claims 8 to 13, designed for the nutrition of pets.

15. Composition according to claim 14 designed for the nutrition of elderly subjects, young subjects in a period of growth and of certain subjects for controlling their body weight.

16. Composition according to claim 14, wherein the source of lipids represents up to 60% of the total energy and the quantity of proteins up to 40% based on the dry extract.

## Patentansprüche

1. Verwendung eines langsam verdaulichen Proteinmaterials für die Herstellung einer Zusammensetzung, die dazu bestimmt ist, Säugern zur Modulierung des postprandialen Aminosäurengehalts des Plasmas auf enteralem Weg verabreicht zu werden, wobei das Proteinmaterial zuvor so behandelt wurde, daß die schnell verdaulichen Proteine, die es enthielt, in langsam verdauliche Proteine überführt werden, **dadurch gekennzeichnet, daß** das langsam verdauliche Proteinmaterial ein Material ist, das gelierte, in Mikropartikel umgewandelte Proteine enthält, die bei Bedingungen der thermodynamischen Unverträglichkeit mit Polysacchariden kombiniert werden.

2. Verwendung eines Proteinmaterials nach Anspruch 1 für die Herstellung einer Zusammensetzung, die dazu dient,
a) den postprandialen Proteingewinn zu erhöhen und/oder
b) eine Stoffwechselüberlastung mancher Organe und/oder mancher Ezyme zu vermeiden und/oder
c) durch eine Sättigungswirkung dieser Proteine die tägliche Nahrungsmitteleinnahme zu reduzieren und/oder
d) gewisse Störungen des Aminosäurestoffwechsels und spezifische Enzymdefizite zu beseitigen
e) die Regeneration von Gewebe, insbesondere die Wundheilungsprozesse, zu verbessern.

3. Verwendung eines Proteinmaterials nach Anspruch 1 für die Herstellung einer Zusammensetzung für die Ernährung von Patienten mit Niereninsuffizienz und/oder hepatitischen Erkrankungen.

4. Verwendung eines Proteinmaterials nach Anspruch 1 für die Herstellung einer Zusammensetzung für die Ernährung von Patienten mit Störungen des Aminosäurestoffwechsels oder spezifischer mit Enzymdefiziten.

5. Verwendung eines Proteinmaterials nach Anspruch 1 für die Herstellung einer Zusammensetzung für die Ernährung von Begleittieren, insbesondere alten Exemplaren, jungen Exemplaren in der Wachstumsperiode und gewissen Exemplaren zur Steuerung ihres Körpergewichts.

6. Verwendung eines Proteinmaterials nach Anspruch 1, **dadurch gekennzeichnet, daß** die Proteine durch thermische Behandlung geliert werden.

7. Verwendung eines Proteinmaterials nach Anspruch 1, **dadurch gekennzeichnet, daß** die Polysaccharide ausgewählt werden aus den Alginaten, Xanthangummi, Gummi arabicum, Guar, Stärke, den Maltodextrinen, den Dextrinen, den Pectinen, den κ-Carageenanen, den 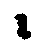 -Carrageenanen, den λ-Carrageenanen, Methylcellulose und Carbomethylcellulose, den sulfatierten Dextranen und/oder Gellangummi.

8. Zusammensetzung zur enteralen Verabreichung an Säuger, die ein langsam verdauliches Proteinmaterial enthält, wobei das Proteinmaterial zuvor so behandelt wurde, daß die schnell verdaulichen Proteine, die es enthielt, in langsam verdauliche Proteine überführt werden, **dadurch gekennzeichnet, daß** das langsam verdauliche Proteinmaterial ein Material ist, das gelierte, in Mikropartikel überführte Proteine enthält, die bei Bedingungen der thermodynamischen Unverträglichkeit mit Polysacchariden kombiniert werden.

9. Zusammensetzung nach Anspruch 8, **dadurch gekennzeichnet, daß** die Polysaccharide ausgewählt werden aus den Alginaten, Xanthangummi, Gummi arabicum, Guar, Stärke, den Maltodextrinen, den Dextrinen, den Pectinen, den κ-Carageenanen, den 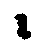 -Carrageenanen, den λ-Carrageenanen, Methylcellulose und Carbomethylcellulose, den sulfatierten Dextranen und/oder Gellangummi.

10. Zusammensetzung nach einem der Ansprüche 8 oder 9, die dazu dient,
a) den postprandialen Proteingewinn zu erhöhen und/oder
b) eine Stoffwechselüberlastung mancher Organe und/oder mancher Ezyme zu vermeiden und/oder
c) durch eine Sättigungswirkung dieser Proteine die tägliche Nahrungsmitteleinnahme zu reduzieren und/oder
d) gewisse Störungen des Aminosäurestoffwechsels und spezifische Enzymdefizite zu beseitigen
e) die Regeneration von Gewebe, insbesondere die Wundheilungsprozesse, zu verbessern.

11. Zusammensetzung nach einem der Ansprüche 8 bis 10, die eine Proteinquelle, die mindestens 8% der Gesamtenergie liefert, eine Glucidquelle, die bis zu 70% der Gesamtenergie liefert, und eine Lipidquelle enthält, die bis zu 35% der Gesamtenergie liefert.

12. Zusammensetzung nach einem der Ansprüche 8 bis 11 mit einer Energiedichte von 70 bis 200 kcal/ml.

13. Zusammensetzung nach einem der Ansprüche 8 bis 12 für Phenylketonurie-Patienten, bei der das Proteinmaterial etwa 50% Caseinoglykomakropeptide, eine Kohlenhydratquelle, eine Lipidquelle und Vitamine und Mineralstoffe enthält.

14. Zusammensetzung nach einem der Ansprüche 8 bis 13 für die Ernährung von Haustieren.

15. Zusammensetzung nach Anspruch 14 für die Ernährung von alten Exemplaren, jungen Exemplaren in der Wachstumsperiode und gewissen Exemplaren zur Steuerung ihres Körpergewichts.

16. Zusammensetzung nach Anspruch 14, bei der die Lipidquelle bis zu 60% der Gesamtenergie und die Proteinmenge bis zu 40% der Trockenmasse darstellt.
